(19) Europäisches Patentamt
European Patent Office
Office européen des brevets

(11) Publication number: **0 201 057 B1**

(12) **EUROPEAN PATENT SPECIFICATION**

(45) Date of publication of patent specification: **02.12.92**

(51) Int. Cl.5: **A61K 31/65**, A61K 31/66

(21) Application number: **86106031.7**

(22) Date of filing: **02.05.86**

The file contains technical information submitted after the application was filed and not included in this specification

# (54) Bone targeted inhibitors of carbonic anhydrase.

(30) Priority: **03.05.85 US 730873**

(43) Date of publication of application:
**12.11.86 Bulletin 86/46**

(45) Publication of the grant of the patent:
**02.12.92 Bulletin 92/49**

(84) Designated Contracting States:
**AT BE CH DE FR GB IT LI LU NL SE**

(56) References cited:
**EP-A- 0 162 510**
**EP-A- 0 195 906**
**US-A- 4 666 897**

**CLINICAL PHARMACY, vol. 4, no. 2, March-April 1985, pages 204-205, Am. Soc. of Hos. Phar. US; D.H. SHEVRIN et al.: "Treatment of cancer-associated hypercalcemia with mithramycin and oral etidronate disodium"**

(73) Proprietor: **RESEARCH CORPORATION TECHNOLOGIES, INC.**
**6840 East Broadway Boulevard**
**Tucson Arizona 85710-2815(US)**

(72) Inventor: **Pierce, William M., Jr.**
**1010 Samuel Street**
**Louisville Kentucky(US)**
Inventor: **Waite, Leonard C.**
**122 Beechmont Drive**
**Corydon Indiana(US)**

(74) Representative: **Hoffmann, Klaus, Dr. rer. nat. et al**
**Hoffmann . Eitle & Partner Patentanwälte**
**Postfach 81 04 20 Arabellastrasse 4**
**W-8000 München 81(DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

PROCEEDINGS OF THE 4th PARATHYROID CONFERENCE 1971, pages 454-462, in: International Congress Series, Excerpta Medica, 1972, Amsterdam, NL; J.J. REYNOLDS: "A sensitive in vivo/in vitro method for studying substances that influence the resorption of bone"

ENDOCRINOLOGY, vol. 87, December 1970, pages 1129-1139; LC. WAITE et al.: "Inhibition of bone resorption by acetazolamide in the rat"

LANCET, May 11, 1974, pages 894-898; R.G.G. RUSSEL et al.: "Disphosphonates in paget's disease"

JOURNAL OF PERIODONTAL RESEARCH, vol. 16, 1981, pages 659-665, Copenhagen, DK; R.C. WILLIAMS et al.: "Tetracycline treatment of periodontal disease in the beagle dog. I. Clinical and radiographic course over 12 months - maximal effect on rate of alveolar bone loss"

ANN. INTERNAL MEDICINE, vol. 96, no. 5, 1982, pages 619-625, Am. Coll. of Physicians, US; S.M. KRANE et al.: "Etidronate disodium in the treatment of paget's disease of bone"

JOURNAL OF PHARMACOLOGY AND EXPERIMENTAL THERAPY, vol. 211, no. 3, 1979, pages 452-455, Am. Soc. for Pharm. and Exp. Therap. US; M.D. LINEBERRY et al.: "Acidosis inhibits the hypocalcemic effect of acetazolamide"

Proc. Soc. Exp. Biol. Med. 186(1987), pages 96-102

## Description

The present invention relates to novel compounds useful in the treatment and prophylaxis of degenerative bone disorders and to a method of their preparation. More specifically, the present invention contemplates novel compounds which are characterized by two active moieties. The first molecular domain possesses "bone-seeking" affinity while the second moiety is a specific inhibitor of the enzyme carbonic anhydrase and/or an inhibitor of bone resorption. The compounds of this invention can be administered alone or as pharmaceutically acceptable compositions and in convenient unit dosage form in a method for the treatment and prophylaxis of degenerative bone disorders.

Bone is a dynamic tissue, consisting of cells in a protein matrix, upon which is superimposed a crystalline structure of various calcium salts. Obviously, the bony skeleton serves as the rigid support for the body. In addition, bone is an organ which responds to hormones. In response to the parathyroid hormone (PTH), bone cells are able to solubilize the calcium salts in bone for use elsewhere in the body. This is a normal regulatory function of bone.

Diseases of excessive bone degeneration exist, including Paget's disease of bone and osteoporosis. The mechanisms are not well understood. In addition, the treatments available are, in general, ad hoc combinations of endocrine and mineral treatments which are often unsuccessful. Clinical osteoporosis is found in approximately 25% of post-menopausal women, and subclinical osteoporosis, which is responsible for untold numbers of bone fractures in elderly women, is far more widespread.

The mechanisms by which bone cells break down bone have been extensively studied but are not clearly defined. One likely scenario is that resorption is caused by the secretion, by bone cells, of acid and proteolytic enzymes. For these enzymes to have their effect, it is likely that the tissue must be decalcified first. Thus, the initiating step is thought to be the acidification of the internal environment of bone, which is responsible for decalcification. One such acid, which has been implicated for many years in these processes, is carbonic acid.

Assuming carbonic acid, which is generated by the enzyme carbonic anhydrase, is involved in bone resorption, then administration of a drug which inhibits carbonic anhydrase should inhibit the liberation of calcium from bone in response to PTH.

This is indeed the case, as was first demonstrated in mammals by Waite, et al. in the publication entitled, "Inhibition of Bone Resorption by Acetazolamide in the Rat", Endocrinology, 87:1129(1970). One of the models used was the Induced Secondary Hyperparathyroid Rat (ISHR). ISHR were prepared by surgical ligation of the renal arteries. In the rat, the kidney is responsible for the metabolism of citrate. Upon ligation of the renal arteries, blood citrate concentration increases. Citrate chelates calcium and, while total calcium concentration is not affected by this binding, the amount of ionized calcium declines. This drop in plasma ionized calcium is the signal for the release of PTH. PTH, once released, signals bone to begin the resorptive process.

As one would predict, in the ISHR this increased release of PTH leads to an increase in total plasma calcium concentration. Administration of the carbonic anhydrase inhibitor acetazolamide to ISHR completely inhibits this response.

Using classic endocrine ablation/replacement studies, it has been shown that this effect is indeed due to a response to PTH. If the ISHR rat has the parathyroid glands removed, the expected increase in plasma calcium concentration is not observed. In this same animal (ISHR without parathyroids) however, administration of PTH evokes the response, while acetazolamide and other heterocyclic sulfonamides (carbonic anhydrase inhibitors) abolish it.

Later work in tissue culture showed that inhibition of PTH-induced resorption by acetazolamide is due to a direct interaction at the level of bone ("Carbonic Anhydrase and Bone Remodeling: Sulfonamide Inhibition of Bone Resorption in Organ Culture", Minkin and Jennings, Science, (June 1970)).

These studies would lead one to suggest that acetazolamide would be useful as an inhibitor of bone resorption. However, when one administers acetazolamide or other heterocyclic sulfonamide carbonic anhydrase inhibitors to normal animals, no change in plasma calcium concentration is observed. It has been demonstrated that the reason for this is that acetazolamide, while inhibiting calcium dissolution from bone due to the PTH response, also causes a systemic acidosis which of itself increases the shift of mineral from bone to blood. These two competing effects mask one another. (See, "Acidosis Inhibits the Hypocalcemic Effect of Acetazolamide", Lineberry and Waite, Pharmacol. Exp. Ther., 211:452 (1979)).

Since these original studies several other factors relative to bone resorption have been determined. For example, the following have subsequently been observed: heterocyclic sulfonamides such as acetazolamide which inhibit carbonic anhydrase also inhibit bone resorption; these sulfonamides have both effects (carbonic anhydrase inhibition and inhibition of bone resorption) at the same concentrations; heterocyclic

sulfonamides which do not inhibit carbonic anhydrase do not inhibit bone resorption; the sulfonamides also inhibit the bone resorptive effects of large doses of Vitamin D; and since other parameters of bone metabolism are not affected by the sulfonamides, this is not a simple toxicity to bone cells. These studies have been accomplished over a period of approximately 15 years and have involved both in vivo and in vitro work.

In view of these prior studies and other additional information recently derived herein, the compounds and methods of the present invention were contemplated in an effort to confer specificity to a novel inhibitor which would be localized specifically in bone so that it would have little or no effect on soft tissue carbonic anhydrase and would be available on site thereby overcoming the inadequacies which presently characterize known regimes for the treatment of degenerative bone disorders.

The present invention relates to a compound for the treatment and prophylaxis of degenerative bone disorders comprising a first moiety which is derived from a bone-seeking agent and a second moiety which is derived from an inhibitor of the enzyme carbonic anhydrase, wherein the compound exhibits in vivo bone-seeking activity and enzyme inhibition activity.

Also, the present invention relates to a method for the preparation of a compound useful for the treatment and prophylaxis of degenerative bone disorders comprising reacting a bone-seeking agent with an inhibitor of the enzyme carbonic anhydrase for a time and under conditions sufficient to form said compound, wherein the compound exhibits in vivo bone-seeking activity and enzyme inhibition activity.

The present invention provides novel compounds which are particularly characterized by two active moieties. The first of these "molecular domains" possesses "bone-seeking" affinity while the second is a specific inhibitor of the enzyme carbonic anhydrase and/or an inhibitor of bone resorption. Optionally, a "bridging" agent can be incorporated into the compounds of the present invention in order to separate the two "active" moieties thereby improving their respective efficacy, i.e., preventing cyclization of these active constituents and avoiding deleterious effects caused by steric hinderance. The present compounds can be prepared by contacting the bone-seeking moiety constituent with the inhibitor constituent for a time and under conditions necessary for the preparation of these novel compounds, conveniently termed "osteostats".

Moreover, the compounds of this invention can be administered alone or in pharmaceutically acceptable compositions in effective amounts sufficient for the treatment and prophylaxis of degenerative bone disorders such as, for example, Paget's disease and osteoporosis.

## BRIEF DESCRIPTION OF THE DRAWINGS

Fig. 1 are graphic illustrations depicting the percentage of acetazolamide, tetracycline, an "osteostat" intermediate and an "osteostat", respectively, which binds to hydroxyapatite (HA) monitored by U.V. spectroscopy at 360 nm.

Fig. 2 are graphic illustrations depicting the results of an assay for carbonic anhydrase activity demonstrating the uncatalyzed reaction time, the enzyme catalyzed reaction time and the enzyme catalyzed reaction time in the presence of acetazolamide, tetracycline, an "osteostat" intermediate and an "osteostat", respectively. These data also demonstrate the activity of the "osteostat" before and after acid hydrolysis.

Fig. 3 is a graphic illustration depicting the relative binding of tetracycline, an "osteostat" intermediate and an "osteostat", respectively, to bone, in vivo.

Fig. 4. is a graphic illustration depicting the plasma calcium concentration in female rats treated with DMSO (control), tetracycline and an "osteostat", respectively.

In accordance with the present invention, novel compounds herein called "osteostats" are provided which are useful for the treatment and prophylaxis of degenerative bone disorders. These compounds are particularly characterized by two active moieties. The first is a compound which exhibits "bone-seeking" affinity. In this context, "bone-seeking" affinity is defined as having the capability to bind calcium with a tendency to accumulate in bone and to incorporate into its crystal lattice. The second necessary constituent of the present compounds is an inhibitor of the enzyme carbonic anhydrase which catalyzes the reversible hydration of carbon dioxide to carbonic acid (and/or an inhibitor of bone resorption). This reaction, as earlier indicated, has been clearly implicated in the bone resorption process which process is defined as the net flux of calcium from bone.

The compounds of the present invention therefore are the reaction products of a bone-seeking compound, an inhibitor of carbonic anhydrase (and/or inhibitor of bone resorption) and, optionally, a bridging agent.

Compounds which exhibit bone-seeking affinity and which exemplify this moiety of the present

compounds are the tetracyclines including, for example, chlortetracycline hydrochloride, demeclocycline hydrochloride, doxycycline, tetracycline, methacycline and oxytetracycline Other bone-seeking moieties within the scope of the present compounds are the diphosphonates such as, for example, ethane-1-hydroxy-1,1-diphosphonic acid (EHDP), dichloromethane diphosphonic acid ($Cl_2$MDP) and 3-amino-1-hydroxypropane-1,1-diphosphonic acid (AHPDP). Tetracycline is preferred.

The carbonic anhydrase inhibitors exemplary of this moiety of the present compounds are the imidazoles, imidazole derivatives and the sulfonamides, particularly acetazolamide, methazolamide, ethoxzolamide and benzolamide acetazolamide being preferred.

Mithramycin is a compound which can be employed in the present invention which exhibits inhibition of bone resorption, but may not be a carbonic anhydrase inhbiitor, per se. Nevertheless, its efficacy is within the scope of the present compounds.

Optionally, although preferably, a third constituent can be incorporated between the other "active" moieties of the present compounds. These "bridging agents" merely insure the efficacy of the active moieties, i.e., by assuring that the compound does not cyclize prior to hydrolysis of the compound in vivo and further avoids the detrimental effects of steric hinderance.

Compounds which are useful as bridging agents in the context of this invention are not limited to any specific group and can, for example, be various $C_4$-$C_{20}$ alkylene chains (difunctional) characterized as having one functional end capable of aromatic carbon electrophilic substitution. Representative of such groups are alkyl halides, alcohols, olefins, esters (i.e., facilitating alkylation in the presence of a Lewis acid), acyl halides, carboxylic acids and anhydrides (i.e., facilitating acylation); and another functional end capable of undergoing an amide forming reaction. Representative of such groups are acids or acid derivatives including, for example, carboxylic acids, anhydrides and acyl halides.

The active moieties, i.e., molecular domains of the "osteostat", can be schematically illustrated as shown below:

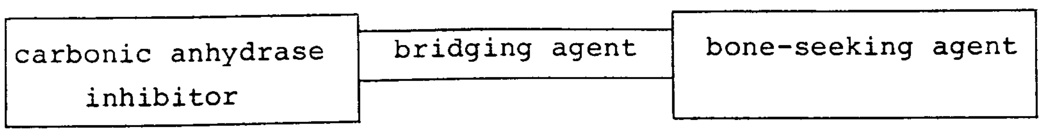


The efficacy of these "osteostats" can be facilitated immediately at the bone site, i.e., having the active sites of both moieties available (not a pro-drug). Preferably, however, the efficacy of these "osteostats" can be facilitated stepwise, first by the bone-seeking moiety which affinity localizes the compound at the bone site. Internally compounded, the osteostat can therefore be a pro-drug, i.e., the active site of the inhibitor is internal in the compound and not immediately available, and will exhibit no initial activity. However, when subjected to the enzymatic hydrolytic conditions occuring at the bone site, i.e., by the production of carbonic acid, the carbonic anhydrase inhibitor will be released in a second step in response to the very process which it is designed to inhibit. This action reflects an ideal feedback system and is elicted only in response to the specific need.

The following scheme of preparation is exemplary of the process which can be employed for the preparation of the present compounds:

sulfonamide + tetracycline or diphosphonate →
(carbonic anhydrase inhibitor) + (bone-seeking agent)

Scheme I

sulfonamide or imidazole — tetracycline or diphosphonate
(osteostat)

A specific example of preparation is shown in Scheme II.

Scheme II

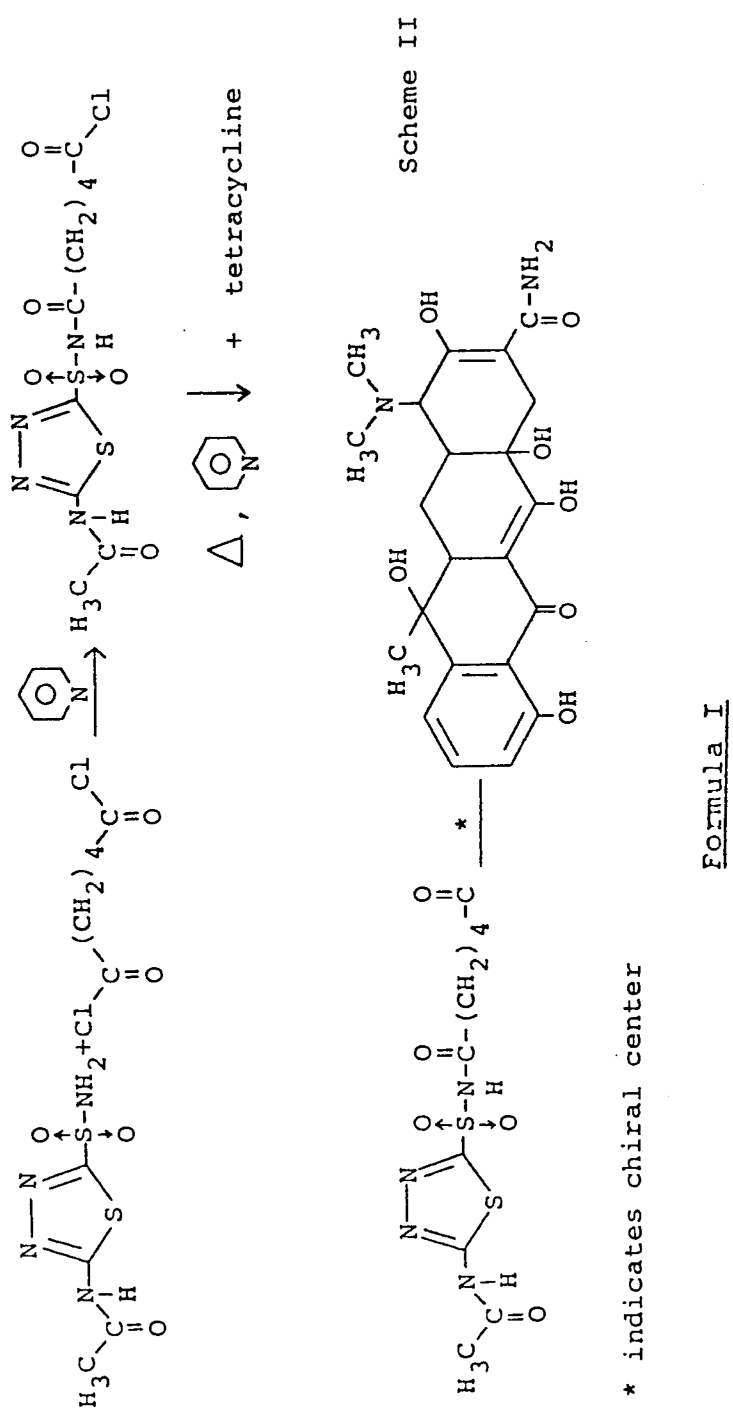


Formula I

The compounds of the present invention may contain one (1) or more asymmetric carbon atoms and may exist in racemic and optically active forms. Depending upon the substituents, the present compounds may form addition salts as well. All of these forms are contemplated to be within the scope of this invention.

The present compounds obviously exist in stereoisomeric forms and the products obtained thus can be mixtures of the isomers, which can be resolved. Alternatively, by selection of specific isomers as starting compounds, the preferred stereoisomer can be produced.

The active ingredients of the therapeutic compositions and the compounds of the present invention exhibit excellent activity in the treatment and prevention of degenerative bone disorders when administered in amounts ranging from about 0.1 mg to about 10 mg per kilogram of body weight per day. A preferred dosage regimen for optimum results would be from about 1 mg to about 10 mg per kilogram of body weight per week, and such dosage units are employed that a total of from about 7 mg to about 700 mg of the

active compound for a subject of about 70 kg of body weight are administered in a 24-hour period. This dosage regimen may be adjusted to provide the optimum therapeutic response and is preferably administered 2 times a day in dosages of about 50 mg per administration. For example, several divided doses may be administered daily or the dose may be proportionally reduced as indicated by the exigencies of the therapeutic situation. A decided practical advantage is that the active compound may be administered in an convenient manner such as by the oral, intraveneous, intramuscular or subcutaneous routes.

The active compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups and wafers. Such compositions and preparations should contain at least 5% of active compound. The percentage of the compositions and preparations may, of course, be varied and may conveniently be between about 1 to about 10% of the weight of the unit. The amount of active compound in such therapeutically useful compositions is such that a suitable dosage will be obtained. Preferred compositions or preparations according to the present invention are prepared so that an oral dosage unit form contains between about 5 and 500 mg of active compound.

The tablets, troches, pills, capsules and the like may also contain the following: A binder such as gum tragacanth, acacia, corn starch or gelatin; excipients; disintegrating agents such as corn starch, potato starch and alginic acid; lubricants; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid and thimerosal. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

As used herein, "pharmaceutically acceptable carrier" includes any and all solvents, dispersion media, coatings, antibacterial and antifungal agents, isotonic and absorption delaying agents. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from about 0.1 to about 1000 mg, with from about 5 to about 500 mg being preferred. Expressed in proportions, the active compound is generally present in from about 1 to about 100 mg/ml of carrier. In the case of compositions containing supplementary active ingredients, the dosages are determined by reference to the usual dose and manner of administration of the said ingredients.

For a better understanding of the present invention together with other and further objects, reference is made to the following description and example.

## EXAMPLE I

### Preparation of the compound of Formula I (reaction product of acetazolamide, adipoyl dichloride and tetracycline)

1 mole of acetazolamide was dissolved in 10% solution of DSMO (wt/v) and added to 2.2 moles of pyridine at room temperature under a nitrogen blanket with stirring. 1 mole of adipoyl dichloride was added dropwise at ambient temperature for one day. One mole of tetracycline (base) was then added and heated to 50 ° C and held for two days.

### Purification

500 ml of 10% slurry of hydroxyapatite in water was added to the preparation and stirred for three hours. The supernatant was decanted washed once with water. 1 mole (aq) EDTA was added in equimolar in ratio with the calcium present in the hydroxyapatite. The pH was adjusted to 3 with HCl and overlaid with 500 ml of N-butyl alcohol and allowed to stand for six hours. The two layers were allowed to separate and then the butyl alcohol was recovered and placed in a rotary evaporator (3 times). Solvent was removed and the drug residue remained: Yield was 15.4% of the theoretical.

For purposes of this experiment, an osteostat intermediate was prepared which was the reaction product of acetazolamide and adipoyl dichloride (an ester).

### Absorption to Hydroxyapatite

To test for the osteostat affinity for bone, test compounds were incubated with a slurry of hydroxyapatite $[Ca_{10}(PO_4)_6OH_2]$, a mineral constituent of bone. The ability of acetazolamide, tetracycline, an osteostat intermediate and the above prepared osteostat to bind to hydroxyapatite crystals, as well as conditions necessary to reverse this binding, were assessed.

Neither acetazolamide nor the intermediate osteostat had any particular affinity for bone. Both tetracycline and the osteostat bound avidly to bone and first indications are that the osteostat has an affinity for bone which is slightly greater than that of tetracycline. This desirable property will permit use at low doses (minimizing side actions) and a high degree of specificity of these agents for bone. The amount of each of these compounds bound was monitored by UV spectroscopy at 360 nm. Data are shown in Fig. 1.

### Inhibition of Carbonic Anhydrase

The assay for carbonic anhydrase activity (see Maren, J. Pharmacol. Exp. Ther., 130:26, 1960) involves determining the rate of acidification of a known solution. Conditions are chosen such that the reaction time in the absence of enzyme (denoted as the uncatalyzed time in Fig. 2) is on the order of 50-60 seconds.

Upon addition of an appropriate amount of carbonic anhydrase, reaction time is reduced to approximately 20 seconds. Test solutions are then added, and prolongation of reaction time indicates the presence of inhibitor.

For each compound, a bar graph is shown in Fig. 2, with data for the uncatalyzed reaction, enzyme catalyzed reaction time and enzyme catalyzed reaction time in the presence of test compounds. Compounds were tested before and after being subjected to mild hydrolytic conditions. Equimolar additions of test compounds were made. Values shown are the average of five determinations plus or minus the standard error of the mean. For each test solution, the solvent used was also tested to assure that the solvent had no effect on reaction time. In no case did the added solvent have any effect.

Delivery of the Osteostat to Bone

Acetazolamide, an intermediate osteostat, tetracycline and the above prepared osteostat were administered to fertilized chicken eggs during periods of rapid growth. On the day before the eggs would have hatched, long bones were harvested from these chicks, the bones were dissolved in dilute aqueous acid (0.1 N HCl for 48 hrs), and this hydrolysate was subjected to U.V. spectroscopy at 360 nm to assess drug deposition. Both tetracycline and the osteostat had been deposited in bone in approximately equimolar amounts. Data are shown in Fig. 3.

In Vivo Inhibition of Bone Resorption

The model used was the Induced Secondary Hyperparathyroid Rat (ISHR). This model has been used for many years and has demonstrated the effects of carbonic anhydrase inhibitors.

In the ISHR, plasma calcium concentration increases over time due to the action of parathyroid hormone on bone. In this study, control animals, as well as animals which received tetracycline, showed the expected increase in plasma calcium concentration. Animals which had been pretreated with an equimolar dose of the prepared osteostat, however, showed no increase in plasma calcium, indicating that the drug is indeed an in vivo inhibitor. See Fig. 4.

These experiments were conducted in Sprague-Dawley derived female rats. Before treatment, their plasma calcium concentration was 11.1 plus or minus 0.3 mg per dL.

Blood samples were taken two hours after ligation of the renal arteries. As always, the control group (which here received an injection of the drug vehicle dimethylsulfoxide) showed a significant increase in plasma calcium concentration. The same increase was seen in tetracycline treated animals. Animals which had received a dose of the osteostat of 50 mg/kg 24 hours prior to surgery showed no increase in plasma calcium. The dose of tetracycline was equimolar with the dose of the osteostat.

Fig. 1

ADSORPTION TO HYDROXYAPATITE

A = Fraction not bound to HA
B = Fraction bound to HA
C = Fraction eluted from HA by 1.0 M phosphate buffer
D = Fraction eluted from HA by 2.0 M phosphate buffer

Fig. 2

INHIBITION OF THE ENZYME CARBONIC ANHYDRASE

The assay for carbonic anhydrase activity (see, Maren, J. Pharmacol. Exp. Ther., 130:26, 1960) involves determining the rate of acidification of a known solution. Conditions are chosen such that the reaction time in the absence of enzyme (denoted as the uncatalyzed time) is on the order of 50-60 seconds. Upon addition of an appropriate amount of carbonic anhydrase, reaction time is reduced to approximately 20 seconds. Test solutions are then added, and prolongation of reaction time indicates the presence of inhibitor.

For each compound, a bar graph is shown, with data for the uncatalyzed reaction, enzyme catalyzed reaction time and enzyme catalyzed reaction time in the presence of test compounds. Compounds were tested before and after being subjected to mild hydrolytic conditions. Equimolar additions of test compounds were made. Values shown are the average of five determinations plus or minus the standard error of the mean. For each test solution, the solvent used was also tested to assure that the solvent had no effect on reaction time. In no case did the added solvent have any effect.

DELIVERY OF DRUG TO BONE IN VIVO

Following harvesting of pretreated chicken bone as described, bones were placed in 0.1 N HCl for 48 hrs. This hydrolysate was then subjected to ultraviolet spectroscopy at a wavelength of 360 nm. This is a characteristic wavelength which is absorbed by tetracycline and the osteostat. Values given are means plus or minus the standard error of the mean.

Fig. 4

INHIBITION OF BONE RESORPTION

These experiments were conducted in Sprague-Dawley derived female rats. Before treatment, their plasma calcium concentration was 11.1 plus or minus 0.3 mg per dL.

Blood samples were taken two hours after ligation of the renal arteries. As always, the control group (which here received an injection of the drug vehicle dimethylsulfoxide) showed a significant increase in plasma calcium concentration. The same increase was seen in tetracycline treated animals. Animals which had received a dose of the osteostat of 50 mg/kg 24 hours prior to surgery showed no increase in plasma calcium. The dose of tetracycline was equimolar with the dose of the osteostat.

**Claims**

**Claims for the following Contracting States : BE, CH, LI, DE, FR, GB, IT, LU, NL, SE**

1. A Compound for the treatment and prophylaxis of degenerative bone disorders comprising a first moiety which is derived from a bone-seeking agent and a second moiety which is derived from an inhibitor of the enzyme carbonic anhydrase, wherein the compound exhibits in vivo bone-seeking activity and enzyme inhibition activity.

2. A composition wherein the compound of claim 1 is in association with a pharmaceutically acceptable carrier.

3. The compound of Claim 1 wherein the inhibitor is mithramycin.

4. The compound of Claim 1 wherein the bone-seeking agent is a tetracycline or a diphosphonate.

5. The compound of Claim 4 wherein the tetracycline is chlortetracycline hydrochloride, demeclocycline hydrochloride, doxycycline, tetracycline, methacycline or oxytetracycline.

6. The compound of Claim 4 wherein the diphosphonate is ethane-1-hydroxy-1,1-diphosphonic acid, dichloromethane diphosphonic acid or 3-amino-1-hydroxypropane-1,1-diphosphonic acid.

7. The compound of Claim 1 wherein said enzyme inhibitor is a heterocyclic sulfonamide or an imidazole.

8. The compound of Claim 7 wherein said sulfonamide is acetazolamide, methazolamide, ethoxzolamide or benzolamide.

9. The compound of claim 1 which further comprises a bridging agent incorporated as a third constituent between the first and second moieties.

10. The compound of Claim 9 wherein said bridging agent is adipoyl dichloride.

11. The composition of Claim 2 in dosage unit form comprising about 5 to about 500 mg of said compound.

12. A method for the preparation of a compound of Claim 1 comprising reacting a bone-seeking agent with

an inhibitor of the enzyme carbonic anhydrase for a time and under conditions sufficient to form said compound, wherein the compound exhibits in vivo bone-seeking activity and enzyme inhibition activity.

**13.** The method of Claim 12 which further comprises first contacting the bone-seeking agent with a bridging agent.

**14.** The method of Claim 13 wherein said bridging agent is adipoyl dichloride.

**15.** The method of any of Claims 12 to 14 wherein the inhibitor is mithramycin.

**16.** The method of any of Claims 12 to 15 wherein the bone-seeking agent is a tetracycline or a diphosphonate.

**17.** The method of Claim 16 wherein the tetracycline is chlortetracycline hydrochloride, demeclocycline hydrochloride, doxycycline, tetracycline, methacycline or oxytetracycline.

**18.** The method of Claim 16 wherein the diphosphonate is ethane-1-hydroxy-1,1-diphosphonic acid, dichloromethane diphosphonic acid or 3-amino-1-hydroxypropane-1,1-diphosphonic acid.

**19.** The method of any of Claims 1 to 18 wherein said enzyme inhibitor is a heterocyclic sulfonamide or an imidazole.

**20.** The method of Claim 19 wherein said sulfonamide is acetazolamide, methazolamide, ethoxzolamide or benzolamide.

**21.** Use of a compound of claim 1 for the preparation of an agent for the treatment and prophylaxis of degenerative bone disorders.

**Claims for the following Contracting State : AT**

**1.** A method for the preparation of a compound for the treatment and prophylaxis of degenerative bone disorders comprising a first moiety which is derived from a bone-seeking agent and a second moiety which is derived from an inhibitor of the enzyme carbonic anhydrase, wherein the compound exhibits in vivo bone-seeking activity and enzyme inhibition activity
comprising reacting a bone-seeking agent with an inhibitor of the enzyme carbonic anhydrase for a time and under conditions sufficient to form said compound, wherein the compound exhibits in vivo bone-seeking activity and enzyme inhibition activity.

**2.** The method of Claim 1 which further comprises first contacting the bone-seeking agent with a bridging agent.

**3.** The method of Claim 2 wherein said bridging agent is adipoyl dichloride.

**4.** The method of any of Claims 1 to 3 wherein the inhibitor is mithramycin.

**5.** The method of any of Claims 1 to 4 wherein the bone-seeking agent is a tetracycline or a diphosphonate.

**6.** The method of Claim 5 wherein the tetracycline is chlortetracycline hydrochloride, demeclocycline hydrochloride, doxycycline, tetracycline, methacycline or oxytetracycline.

**7.** The method of Claim 5 wherein the diphosphonate is ethane-1-hydroxy-1,1-diphosphonic acid, dichloromethane diphosphonic acid or 3-amino-1-hydroxypropane-1,1-diphosphonic acid.

**8.** The method of any of Claims 1 to 7 wherein said enzyme inhibitor is a heterocyclic sulfonamide or an imidazole.

**9.** The method of Claim 8 wherein said sulfonamide is acetazolamide, methazolamide, ethoxzolamide or

benzolamide.

10. Use of a compound comprising the reaction product of a bone-seeking agent and an inhibitor of the enzym carbonic anhydrase for the preparation of an agent for the treatment and prophylaxis of degenerative bone disorders.

**Patentansprüche**
**Patentansprüche für folgende Vertragsstaaten : BE, CH, LI, DE, FR, GB, IT, LU, NL, SE**

1. Verbindung für die Behandlung und Prophylaxe von degenerativen Knochenerkrankungen, umfassend eine erste Komponente, die sich von einem knochensuchenden Mittel ableitet und eine zweite Komponente, die sich von einem Inhibitor des Enzyms Carboanhydrase ableitet, wobei die Verbindung in vivo eine knochensuchende Aktivität und eine Enzyminhibierungsaktivität aufweist.

2. Zusammensetzung, in welcher die Verbindung des Anspruchs 1 zusammen mit einem pharmazeutische annehmbaren Träger vorliegt.

3. Verbindung gemäß Anspruch 1, worin der Inhibitor Mithramycin ist.

4. Verbindung gemäß Anspruch 1, worin die knochensuchende Verbindung ein Tetracyclin oder ein Diphosphonat ist.

5. Verbindung gemäß Anspruch 4, in welcher das Tetracyclin Chlortetracyclin-Hydrochlorid, Demecyklocyclin-Hydrochlorid, Doxycyclin, Tetracyclin, Metacyclin oder Oxitetracyclin ist.

6. Verbindung gemäß Anspruch 4, in welcher das Diphosphonat Ethan-1-hydroxy-1,1-diphosphonsäure, Dichlormethandiphosphonsäure oder 3-Amino-1-hydroxypropan-1,1-diphosphonsäure ist.

7. Verbindung gemäß Anspruch 1, in welcher der Enzyminhibitor ein heterocyclisches Sulfonamid oder ein Imidazol ist.

8. Verbindung gemäß Anspruch 7, in welcher das Sulfonamid Acetazolamid, Methazolamid, Ethoxzolamid oder Benzolamid ist.

9. Verbindung gemäß Anspruch 1, welche weiterhin ein Überbrückungsmittel, das als ein dritter Bestandteil zwischen der ersten und der zweiten Komponente inkorporiert ist, einschließt.

10. Verbindung gemäß Anspruch 1, in welcher das Überbrückungsmittel Adipoyldichlorid ist.

11. Verbindung gemäß Anspruch 2 in Einheitsdosierungsform, umfassend etwa 5 bis etwa 500 mg der Verbindung.

12. Verfahren zum Herstellen einer Verbindung gemäß Anspruch 1, umfassend das Umsetzen eines knochensuchenden Mittels mit einem Inhibitor des Enzyms Carboanhydrase für einen Zeitraum und unter Bedingungen, die ausreichen, um die Verbindung zu bilden, wobei die Verbindung in vivo knochensuchende Aktivität und Enzyminhibierungsaktivität aufweist.

13. Verfahren gemäß Anspruch 12, welches weiterhin umfaßt, daß man zunächst das knochensuchende Mittel mit einem Überbrückungsmittel kontaktiert.

14. Verfahren gemäß Anspruch 13, in welchem das Überbrückungsmittel Adipoyldichlorid ist.

15. Verfahren gemäß einem der Ansprüche 12 bis 14, bei dem der Inhibitor Mithramycin ist.

16. Verfahren gemäß einem der Ansprüche 12 bis 15, bei dem das knochensuchende Mittel ein Tetracyclin Oder ein Diphosphonat ist.

17. Verfahren gemäß Anspruch 16, bei dem das Tetracyclin Chlortetracyclin-Hydrochlorid, Demeclocyclin-Hydrochlorid, Doxycyclin, Tetracyclin, Methacyclin oder Oxytetracyclin ist.

**18.** Verfahren gemäß Anspruch 16, bei dem das Diphosphonat Ethan-1-hydroxy-1,1-diphosphonsäure, Dichlormethan-Diphosphonsäure oder 3-Amino-1-hydroxypropan-1,1-diphosphonsäure ist.

**19.** Verfahren gemäß einem der Ansprüche 1 bis 18, bei dem der Enyminhibitor ein heterocyclisches Sulfonamid oder ein Imidazol ist.

**20.** Verfahren gemäß Anspruch 19, bei dem das Sulfonamid Acetazolamid, Methazolamid, Ethoxzolamid oder Benzolamid ist.

**21.** Verwendung einer Verbindung gemäß Anspruch 1 zur Herstellung eines Mittels für die Behandlung und Prophylaxe von degenerativen Knochenerkrankungen.

**Patentansprüche für folgenden Vertragsstaat : AT**

**1.** Verfahren zur Herstellung einer Verbindung für die Behandlung und Prophylaxe von degenerativen Knochenerkrankungen, umfassend eine erste Komponente, die sich von einem knochensuchenden Mittel ableitet und eine zweite Komponente, die sich von einem Inhibitor des Enzyms Carboanhydrase ableitet, wobei die Verbindung in vivo knochensuchende Aktivität und Enzyminhibierungsaktivität aufweist, umfassend das Umsetzen eines knochensuchenden Mittels mit einem Inhibitor des Enzyms Carboanhydrase während einer Zeit und unter Bedingungen, die ausreichen, um die Verbindung zu bilden, wobei die Verbindung in vivo knochensuchende Aktivität und Enzyminhibierungsaktivität aufweist.

**2.** Verfahren gemäß Anspruch 1, umfassend weiterhin, daß man zunächst das knochensuchende Mittel mit einem Überbrückungsmittel kontaktiert.

**3.** Verfahren gemäß Anspruch 2, bei dem das Überbrückungsmittel Adipoyldichlorid ist.

**4.** Verfahren gemäß einem der Ansprüche 1 bis 3, bei dem der Inhibitor Mithramycin ist.

**5.** Verfahren gemäß einem der Ansprüche 1 bis 4, bei dem das knochensuchende Mittel ein Tetracyclin oder ein Diphosphonat ist.

**6.** Verfahren gemäß Anspruch 5, bei dem das Tetracyclin Chlortetracyclin-Hydrochlorid, Demeclocyclin-Hydrochlorid, Doxycyclin, Tetracyclin, Methacyclin oder Oxitetracyclin ist.

**7.** Verfahren gemäß Anspruch 5, bei dem das Diphosphonat Ethan-1-hydroxy-1,1-diphosphonsäure, Dichlormethandiphosphonsäure oder 3-Amino-1-hydroxypropan-1,1-diphosphonsäure ist.

**8.** Verfahren gemäß einem der Ansprüche 1 bis 7, bei dem der Enzyminhibitor ein heterocyclisches Sulfonamid oder ein Imidazol ist.

**9.** Verfahren gemäß Anspruch 8, bei dem das Sulfonamid Acetazolamid, Methazolamid, Ethoxzolamid oder Benzolamid ist.

**10.** Verwendung einer Verbindung umfassend das Umsetzungsprodukt eines knochensuchenden Mittels und eines Inhibitors des Enzyms Carboanhydrase zur Herstellung eines Mittels für die Behandlung und Prophylaxe von degenerativen Knochenerkrankungen.

**Revendications**
**Revendications pour les Etats contractants suivants : BE, CH, LI, DE, FR, GB, IT, LU, NL, SE**

**1.** Composé pour le traitement et la prophylaxie de désordres osseux dégénératifs, comprenant une première fraction qui est issue d'un agent ostéotrope et une seconde fraction qui est issue d'un inhibiteur de l'enzyme anhydrase carbonique, le composé manifestant une activité ostéotrope in vivo et une activité d'inhibition d'enzyme in vivo.

2. Composition dans laquelle le composé de la revendication 1 est en association avec un support pharmaceutiquement acceptable.

3. Composé selon la revendication 1, dans lequel l'inhibiteur est la mithramycine.

4. Composé selon la revendication 1, dans lequel l'agent ostéotrope est une tétracycline ou un diphosphonate.

5. Composé selon la revendication 1, dans lequel la tétracycline est le chlorhydrate de chlorotétracycline, le chlorhydrate de déméclocycline, la doxycycline, la tétracycline, la méthacycline ou l'oxytétracycline.

6. Composé selon la revendication 4, dans lequel le diphosphonate est l'acide éthane-hydroxy-1 diphosphonique-1,1, l'acide dichlorométhane diphosphonique ou l'acide amino-3 hydroxy-1 propane diphosphonique-1,1.

7. Composé selon la revendication 1, dans lequel ledit inhibiteur d'enzyme est un sulfonamide hétérocyclique ou un imidazole.

8. Composé selon la revendication 7, dans lequel ledit sulfonamide est l'acétazolamide, le méthazolamide, l'éthoxzolamide ou le benzolamide.

9. Composé selon la revendication 1, qui comprend en outre un agent de pontage incorporé sous la forme d'un troisième constituant entre les première et seconde fractions.

10. Composé selon la revendication 9, dans lequel ledit agent de pontage est le dichlorure d'adipoyle.

11. Composition selon la revendication 2 se présentant sous la forme d'unités de dosage comprenant environ 5 à environ 500 mg dudit composé.

12. Procédé pour la préparation d'un composé tel que défini à la revendication 1, comprenant la réaction d'un agent ostéotrope avec un inhibiteur de l'enzyme anhydrase carbonique pendant un laps de temps suffisant et dans des conditions suffisantes pour former ledit composé, le composé manifestant une activité ostéotrope in vivo et une activité d'inhibition d'enzyme in vivo.

13. Procédé selon la revendication 12, qui comprend en outre l'opération consistant à mettre d'abord en contact l'agent ostéotrope avec un agent de pontage.

14. Procédé selon la revendication 13, dans lequel ledit agent de pontage est le dichlorure d'adipoyle.

15. Procédé selon l'une quelconque des revendications 12 à 14, dans lequel l'inhibiteur est la mithramycine.

16. Procédé selon l'une quelconque des revendications 12 à 15, dans lequel l'agent ostéotrope est une tétracycline ou un diphosphonate.

17. Procédé selon la revendication 16, dans lequel la tétracycline est le chlorhydrate de chlorotétracycline, le chlorhydrate de déméclocycline, la doxycycline, la tétracycline, la méthacycline ou l'oxytétracycline.

18. Procédé selon la revendication 16, dans lequel le diphosphonate est l'acide éthane-hydroxy-1 diphosphonique-1,1, l'acide dichlorométhane diphosphonique ou l'acide amino-3 hydrory-1 propane diphosphonique-1,1.

19. Procédé selon l'une quelconque des revendications 1 à 18, dans lequel ledit inhibiteur d'enzyme est un sulfonamide hétérocyclique ou un imidazole.

20. Procédé selon la revendication 19, dans lequel ledit sulfonamide est l'acétazolamide, le méthazolamide, l'éthoxzolamide ou le benzolamide.

21. Utilisation d'un composé tel que défini à la revendication 1 pour la préparation d'un agent pour le traitement et la prophylaxie de désordres osseux dégénératifs.

**Revendications pour l'Etat contractant suivant : AT**

1. Procédé de préparation d'un composé pour le traitement et la prophylaxie de désordres osseux dégénératifs, comprenant une première fraction qui est issue d'un agent ostéotrope et une seconde fraction qui est issue d'un inhibiteur de l'enzyme anhydrase carbonique, le composé manifestant une activité ostéotrope in vivo et une activité d'inhibition d'enzyme in vivo,
comprenant la réaction d'un agent ostéotrope avec un inhibiteur de l'enzyme anhydrase carbonique pendant un laps de temps suffisant et dans des conditions suffisantes pour former ledit composé, le composé manifestant une activité ostéotrope in vivo et une activité d'inhibition d'enzyme in vivo.

2. Procédé selon la revendication 1, qui comprend en outre l'opération consistant à mettre d'abord en contact l'agent ostéotrope avec un agent de pontage.

3. Procédé selon la revendication 2, dans lequel ledit agent de pontage est le dichlorure d'adipoyle.

4. Procédé selon l'une quelconque des revendications 1 à 3, dans lequel l'inhibiteur est la mithramycine.

5. Procédé selon l'une quelconque des revendications l à 4, dans lequel l'agent ostéotrope est une tétracycline ou un diphosphonate.

6. Procédé selon la revendication 5, dans lequel la tétracycline est le chlorhydrate de chlorotétracycline, le chlorhydrate de déméclocycline, la doxycycline, la tétracycline, la méthacycline ou l'oxytétracycline.

7. Procédé selon la revendication 5, dans lequel le diphosphonate est l'acide éthane-hydroxy-1 diphosphonique-1,1, l'acide dichlorométhane diphosphonique ou l'acide amino-3 hydroxy-1 propane diphosphonique-1,1.

8. Procédé selon l'une quelconque des revendications 1 à 7, dans lequel ledit inhibiteur d'enzyme est un sulfonamide hétérocyclique ou un imidazole.

9. Procédé selon la revendication 8, dans lequel ledit sulfonamide est l'acétazolamide, le méthazolamide, l'éthoxzolamide ou le benzolamide.

10. Utilisation d'un composé comprenant le produit de réaction d'un agent ostéotrope et d'un inhibiteur de l'enzyme anhydrase carbonique pour la préparation d'un agent pour le traitement et la prophylaxie de désordres osseux dégénératifs.

FIG. 1

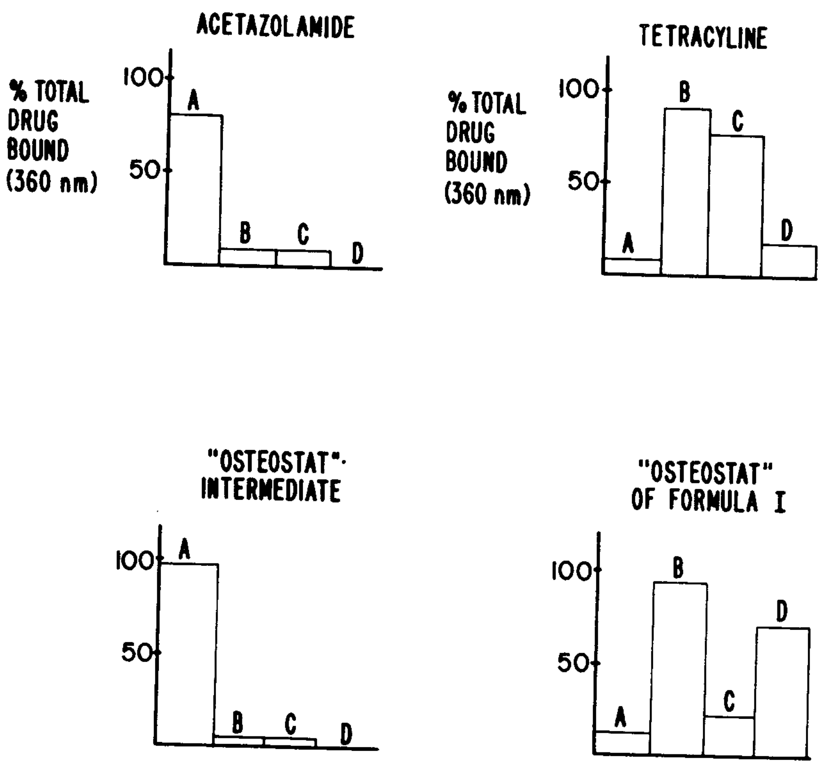
ACETAZOLAMIDE
% TOTAL DRUG BOUND (360 nm)
100
50
A
B
C
D
TETRACYLINE
% TOTAL DRUG BOUND (360 nm)
100
50
A
B
C
D
"OSTEOSTAT"-INTERMEDIATE
100
50
A
B
C
D
"OSTEOSTAT" OF FORMULA I
100
50
A
B
C
D

FIG. 2

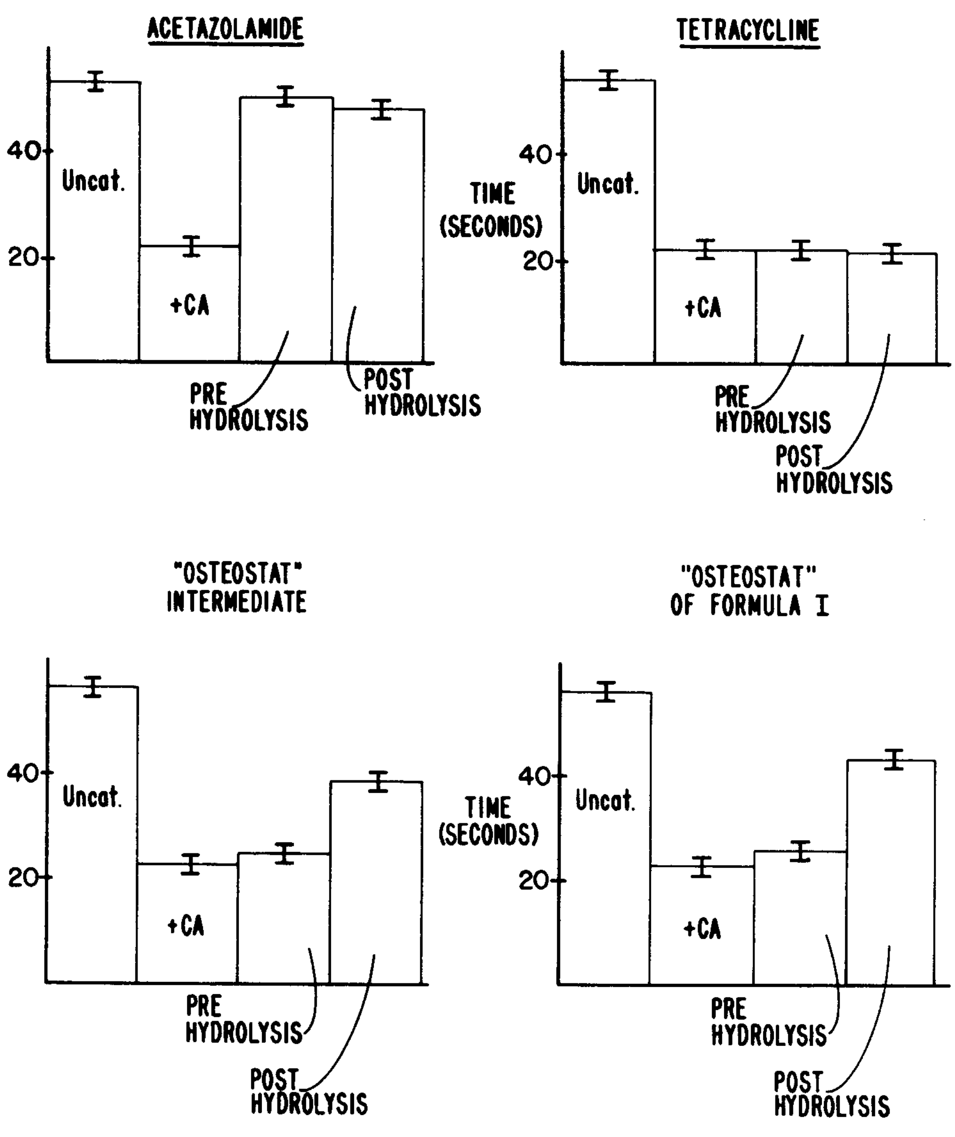
ACETAZOLAMIDE
TETRACYCLINE
"OSTEOSTAT" INTERMEDIATE
"OSTEOSTAT" OF FORMULA I
TIME (SECONDS)
40
20
Uncat.
+CA
PRE HYDROLYSIS
POST HYDROLYSIS

FIG. 3

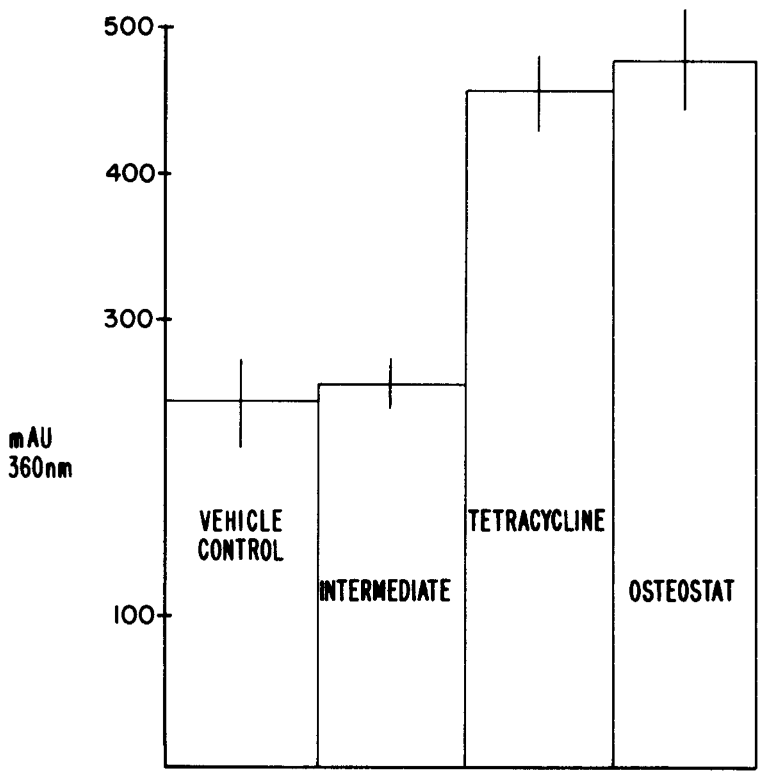
500
400
300
100
mAU
360nm
VEHICLE
CONTROL
INTERMEDIATE
TETRACYCLINE
OSTEOSTAT

FIG. 4

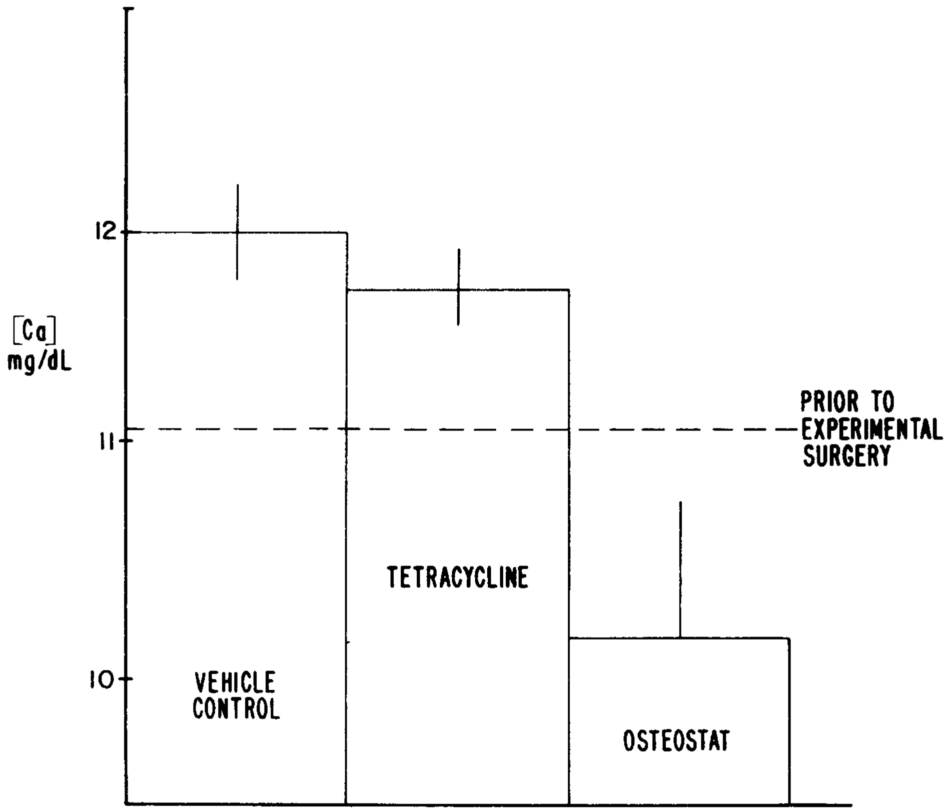
12
[Ca]
mg/dL
11
10
PRIOR TO
EXPERIMENTAL
SURGERY
VEHICLE
CONTROL
TETRACYCLINE
OSTEOSTAT